# EUROPÄISCHE PATENTSCHRIFT

(11) **EP 4 584 773 B1**
(45) Veröffentlichungstag und Bekanntmachung des Hinweises auf die Patenterteilung: **19.11.2025**
(21) Anmeldenummer: 24790283.6
(22) Anmeldetag: 23.09.2024
(51) Int. Cl.: G09B 19/00, A61F 11/00

(54) **VORRICHTUNG UND SYSTEM ZUM ERLERNEN, TRAINIEREN UND ZUR DURCHFÜHRUNG EINES DRUCKAUSGLEICHS IM MITTELOHR**
DEVICE AND SYSTEM FOR LEARNING, TRAINING AND CARRYING OUT A PRESSURE COMPENSATION IN THE MIDDLE EAR
DISPOSITIF ET SYSTÈME D'APPRENTISSAGE, D'ENTRAÎNEMENT ET DE RÉALISATION D'UNE COMPENSATION DE PRESSION DANS L'OREILLE INTERNE

(30) Priorität: 26.09.2023 AT 601742023
(43) Veröffentlichungstag der Anmeldung: 16.07.2025
(73) Patentinhaber: EARBREEZE GMBH, 1010 Wien (AT)
(72) Erfinder: SCHRÖCKENFUCHS, Michael, 2340 Mödling (AT); KURSCHEL, Martin, 1010 Wien (AT)
(74) Vertreter: Schardmüller Gall-Schuhmann Patentanwälte OG
(86) Internationale Anmeldenummer: PCT/EP2024/076639
(87) Internationale Veröffentlichungsnummer: WO 2025/073527

(56) Entgegenhaltungen:
- IT-A1- UB20 156 864
- US-A- 5 485 832
- US-A- 5 950 631
- US-A1- 2008 196 714

## Beschreibung

### Gebiet der Erfindung

Die vorliegende Erfindung betrifft eine Vorrichtung zur Durchführung eines Druckausgleichs zwischen einem Mittelohr eines Benutzers und einer Umgebung des Mittelohrs.

Weiters betrifft die Erfindung ein System umfassend die erfindungsgemäße Vorrichtung sowie lösbar damit verbundene Nasenstopfen.

### Stand der Technik

Insbesondere beim Tauchen, Liftfahren und Fliegen (Steig-/Sinkflug) entsteht im Mittelohr ein Unter- bzw. Überdruck im Vergleich zur Umgebung (Wasser-, Luft- oder Kabinendruck). Auch Auto- und Motorradreisen über Bergstraßen, Zugluft und Klimaanlagen, chronische Entzündungen in der Nase, Allergien, Erkältungen, anatomische Engstellen und verstopfte Ohren (Cerumen obturans) können zu einer entsprechenden Druckdifferenz führen.

Da das Mittelohr durch die Eustachische Röhre (auch Eustachi-Röhre, Eustachiröhre, Eustachi'sche Röhre oder Ohrtrompete, lat.-anat. Tuba auditiva Eustachii oder Tuba pharyngotympanica) mit dem Rachen verbunden ist, kann über diese - auch Tube genannte - Verbindung ein Druckausgleich zwischen Mittelohr und der Umgebung herbeigeführt werden.

Die Tube ist im Normalfall verschlossen; die Fähigkeit dieses normalerweise verschlossenen Kanals sich zu öffnen, ist individuell sehr verschieden. Beim gesunden Ohr sorgen bereits Schlucken und Gähnen dafür, dass sich die Tube öffnet und ein Druckausgleich herbeigeführt werden kann. Alternativ kann eine Öffnung der Tube und in weiterer Folge auch der Druckausgleich durch Ausatmen in die zugehaltene Nase erreicht werden; diese Form des Druckausgleichs ist auch als Valsalva-Versuch bekannt. Hilft auch diese Maßnahme nicht, kann das ein Hinweis auf eine Tubenbelüftungsstörung sein. In Extremfällen kann ein nicht erfolgreicher Druckausgleich zu starken Ohren- und Kopfschmerzen und letzten Endes zu einem Barotrauma führen.

Es zeigt sich also, dass einige Menschen praktisch nie Druckausgleichsprobleme haben, während andere Menschen, beispielsweise aufgrund einer besonders engen oder teilweise blockierten Eustachischen Röhre, insbesondere beim Tauchen, Fliegen oder Liftfahren Schwierigkeiten haben, den Druck im Mittelohr auszugleichen; bei ihnen verlangt die Durchführung des Druckausgleichs mehr Aufmerksamkeit und viel Übung.

Wenn herkömmliche Methoden scheitern, kann die sogenannte Edmonds-Technik zum Druckausgleich angewendet werden; dabei wird durch eine spezielle Kieferbewegung eine leichte Streckung der Eustachischen Röhre bewirkt und in Kombination mit der Valsalva-Technik (oder der damit verwandten Frenzel-Methode) der Druckausgleich herbeigeführt. Der hierzu notwendige Bewegungsablauf stellt sich aber als durchaus komplex dar und muss in der Regel mühsam erlernt werden.

### Aufgabe der Erfindung

Eine Aufgabe der vorliegenden Erfindung besteht daher darin, eine Vorrichtung zum Erlernen, Trainieren und Durchführen einer Methode zur Herbeiführung eines Druckausgleichs im Mittelohr bereitzustellen.

Weitere Aufgaben ergeben sich aus der vorliegenden Beschreibung.

### Darstellung der Erfindung

Eine Aufgabe der Erfindung wird gelöst durch eine Vorrichtung zur Durchführung eines Druckausgleichs zwischen einem Mittelohr eines Benutzers und einer Umgebung des Mittelohrs, wobei die Vorrichtung zumindest einen Beißabschnitt zur Aufnahme zumindest eines Abschnitts einer unteren Zahnreihe des Benutzers, zumindest einen Nasenabschnitt zum Verschließen einer Nase des Benutzers sowie zumindest einen Betätigungsabschnitt zur manuellen Betätigung der Vorrichtung durch den Benutzer zur Durchführung des Druckausgleichs aufweist, wobei Beißabschnitt, Nasenabschnitt und Betätigungsabschnitt voneinander beabstandet und kräfteübertragend miteinander verbunden sind, und wobei der Beißabschnitt zumindest ein erstes Rückhalteelement aufweist, um einen Anschlag für die untere Zahnreihe des Benutzers auszubilden.

Die erfindungsgemäße Vorrichtung zeichnet sich demnach zunächst dadurch aus, dass der Beißabschnitt, der Nasenabschnitt und der Betätigungsabschnitt voneinander beanstandet und kräfteübertragen miteinander verbunden sind. Dadurch wird es möglich, durch Ausübung einer (manuellen) Kraft auf den Betätigungsabschnitt sowohl den Beißabschnitt als auch den Nasenabschnitt gleichzeitig mit einer Kraft zu beaufschlagen, wodurch einerseits eine spezielle Relativbewegung zwischen Unter- und Oberkiefer des Benutzers ausgeführt und andererseits gleichzeitig die Nase des Benutzers verschlossen wird. Atmet der Benutzer nun gegen die (verschlossene) Nase aus, kommt es zu einem Druckausgleich.

In einem Betriebszustand der Vorrichtung, in dem die Vorrichtung vom Benutzer bestimmungsgemäß zur Durchführung des Druckausgleichs im Mittelohr verwendet wird, befindet sich der Beißabschnitt in Kontakt mit der unteren Zahnreihe, vorzugsweise mit den unteren Schneidezähnen (Kennziffern 31 und 41 nach dem FDI-Zahnschema), des Benutzers. Dabei kann das Rückhalteelement bereits an der zungenseitigen Innenseite der unteren Zahnreihe anliegen oder hinter dieser Innenseite - also tiefer in der Mundhöhle des Benutzers - positioniert sein. Vorzugsweise beißt der Benutzer sowohl mit Zähnen der oberen als auch mit Zähnen der unteren Zahnreihe (locker) auf den Beißabschnitt der Vorrichtung. Anschließend kann der Benutzer die Vorrichtung mit einer Hand, beispielsweise mit zwei Fingern, am Betätigungsabschnitt halten und den Betätigungsabschnitt mit einer Kraft beaufschlagen. Dazu kann der Benutzer den Betätigungsabschnitt nach oben, in Richtung seiner Stirn, anheben, was dazu führt, dass sich der Nasenabschnitt in Richtung der Nasenlöcher des Benutzers bewegt. Sobald der Nasenabschnitt der Vorrichtung (oder mit dem Nasenabschnitt verbundene Nasenstopfen, die weiter unten beschrieben sind) an dem Nasenloch bzw. den Nasenlöchern anliegt, kommt es zu einem Verschluss der Nase. Dieser Verschluss kann zunächst noch durch festes Ausatmen gegen den Nasenabschnitt (oder gegen die Nasenstopfen) überwunden werden, sodass Luft aus den Nasenlöchern - vorbei an dem Nasenabschnitt - ausströmen kann. Um die Nase fester zu verschließen, kann der Benutzer in weiterer Folge noch ein wenig mehr Kraft auf den Betätigungsabschnitt ausüben, sodass der Nasenabschnitt (oder die Nasenstopfen) fest an den Nasenlöchern des Benutzers anliegt. Nun verhindert der Nasenabschnitt bzw. die Nasenstopfen ein Ausströmen von Luft aus der Nase. Die vom Benutzer auf den Betätigungsabschnitt ausgeübte Kraft führt nun - da die Vorrichtung über den Nasenabschnitt an der Nase des Benutzers gelagert ist - zu einer Rotation der Vorrichtung, durch welche Rotation der Beißabschnitt eine (im Wesentlichen) horizontale Distanz in eine vom Körper des Benutzers weg weisende Richtung zurücklegt, die durch eine (im Wesentlichen) vertikale Distanz bestimmt ist, um welche vertikale Distanz der Benutzer den Betätigungsabschnitt anhebt. Durch diese horizontale Bewegung des Beißabschnittes wird das Unterkiefer - im Wesentlichen parallel zum Oberkiefer - verschoben, sodass sich ein Unterbiss ausbildet. In anderen Worten: Der Beißabschnitt führt die untere Zahnreihe des Benutzers mittels des Rückhalteelementes vor dessen obere Zahnreihe, wobei die Bewegung des Beißabschnittes durch das Rückhalteelement, das als Anschlag für die am Beißabschnitt befindlichen Zähne der unteren Zahnreihe des Benutzers fungiert, auf die untere Zahnreihe des Benutzers übertragen wird. In dieser Position, in der der Unterkiefer nach vorne geführt und die Nase zumindest so fest verschlossen ist, dass bei mäßig bis starkem Ausatmen in die Nase keine Luft aus den Nasenlöchern entweichen kann, führt ein Atmen gegen die verschlossene Nase zu einem Druckausgleich im Mittelohr. Dies ist darauf zurückzuführen, dass der Benutzer die Edmonds-Technik angewendet und die dabei vorgesehene Bewegungs- und Atmungsabfolge durchgeführt hat.

Durch die Erfindung wird es somit möglich, den durchaus komplexen Bewegungsablauf der Edmonds-Technik auf ein Aufbeißen auf den Beißabschnitt, ein nach oben Drücken des Betätigungsabschnittes und ein Ausatmen gegen die durch den Nasenabschnitt (oder die Nasenstopfen) verschlossene Nase (und vorzugsweise auch bei geschlossenem Mund) reduziert. Die erfindungsgemäße Vorrichtung eignet sich somit einerseits zur Durchführung bzw. Herbeiführung eines Druckausgleichs im Mittelohr (im Akutfall), andererseits aber auch zum Erlernen der erforderlichen Bewegungsabfolge sowie zum Trainieren der Edmonds-Technik.

Gemäß einer bevorzugten Ausführungsform der erfindungsgemäßen Vorrichtung ist es vorgesehen, dass Beißabschnitt, Nasenabschnitt und Betätigungsabschnitt über eine Gabelung miteinander verbunden sind.

Hierdurch lässt sich eine besonders einfache und gleichzeitig besonders effiziente Struktur der erfindungsgemäßen Vorrichtung realisieren. Insbesondere kann der Beißabschnitt - unabhängig von der Ausbildung des Nasenabschnittes und des Betätigungsabschnittes - besonders zielgerichtet zur Aufnahme der unteren (und vorzugsweise auch der oberen) Zahnreihe des Patienten ausgebildet werden; der Nasenabschnitt - unabhängig vom Beißabschnitt und vom Betätigungsabschnitt - zum Verschließen der Nase ausgebildet werden; und der Betätigungsabschnitt - unabhängig vom Beißabschnitt und vom Betätigungsabschnitt - besonders ergonomisch ausgebildet werden. Die Gabelung wiederum kann insbesondere durch eine strukturelle Verstärkung ausgebildet sein oder eine solche umfassen, um eine zuverlässige Übertragung, insbesondere auch von hohen, (manuell aufgebrachten) Kräften sicherzustellen.

Gemäß einer bevorzugten Ausführungsform der erfindungsgemäßen Vorrichtung ist es vorgesehen, dass die Vorrichtung einen gabelartig ausgebildeten Hebel umfasst oder als gabelartiger Hebel ausgebildet ist, wobei der Beißabschnitt an einem ersten Hebelarm, der Nasenabschnitt an einem zweiten Hebelarm und der Betätigungsabschnitt an einem dritten Hebelarm angeordnet ist.

Dadurch wird es möglich, den den Nasenabschnitt aufweisenden Hebelarm in anatomisch besonders günstiger Weise auszuformen, um diesen möglichst nahe an den Nasenlöchern des Benutzers zu positionieren, wenn der Benutzer die Vorrichtung über den Beißabschnitt klemmend zwischen Ober- und Unterkiefer hält. Gleichzeitig kann der den Beißabschnitt aufweisende Hebelarm so ausgeführt sein, dass das Rückhalteelement derart im bzw. am Beißabschnitt positioniert ist bzw. derart ausgebildet ist, dass eine möglichst effiziente Führung des Unterkiefers in die gewünschte Stellung gewährleistet ist, ohne eine Beschädigung der Zähne des Benutzers zu riskieren. Der Betätigungsabschnitt wiederum kann so ausgeführt sein, dass eine besonders einfache Bedienung der Vorrichtung ermöglicht ist und die vom Benutzer aufzubringende Kraft möglichst gering ist.

Gemäß einer bevorzugten Ausführungsform der erfindungsgemäßen Vorrichtung ist es vorgesehen, dass die Vorrichtung einen Steg umfasst, wobei Beißabschnitt und Betätigungsabschnitt durch jeweils einen Abschnitt, vorzugsweise Endabschnitt, des Steges ausgebildet sind.

Der Steg ermöglicht einerseits einen möglichst sicheren Halt des Beißabschnittes zwischen oberer und unterer Zahnreihe des Benutzers, da die zur Aufnahme von Zähnen der unteren und gegebenenfalls auch der oberen Zahnreihe zur Verfügung stehende Auflagefläche im Bereich des Beißabschnittes vergrößert werden kann (ähnlich einer Beißschiene). Andererseits lässt sich dadurch aber auch die Handhabung der Vorrichtung durch den Benutzer angenehmer gestalten, da die Klemmung eines (flachen) Stegs zwischen Ober- und Unterkiefer einen vergleichsweise kleinen Öffnungswinkel erfordert. Zudem ermöglicht die Ausbildung der Vorrichtung als stegförmiges Element bzw. als Steg, insbesondere im Bereich des Beißabschnittes, ein lockeres und einfaches Schließen des Mundes um den Steg, wodurch sich komplexe Bewegungsabfolge noch einfacher und natürlicher erlernen, trainieren und durchführen lässt.

Gemäß einer bevorzugten Ausführungsform der erfindungsgemäßen Vorrichtung ist es vorgesehen, dass die Vorrichtung eine Halterung umfasst, welche Halterung von dem Steg abragt und den Nasenabschnitt aufweist.

Steg und Halterung können dabei durchaus einstückig ausgebildet sein; allerdings ermöglicht die gesonderte Ausbildung der Halterung eine besonders günstige Anpassung der erfindungsgemäßen Vorrichtung an die Physiognomie des menschlichen Gesichtes. Insbesondere kann die Halterung - im Vergleich zu anderen Bereichen der Vorrichtung - besonders filigran und materialsparend ausgebildet sein, wodurch sich die Fertigungskosten der erfindungsgemäßen Vorrichtung reduzieren lassen. Zudem kann die Vorrichtung dadurch - was für die Akzeptanz von Hygieneartikeln, medizinischen Hilfsmitteln und Heilbehelfen nicht unwichtig ist - auch optisch ansprechend gestaltet werden.

Gemäß einer bevorzugten Ausführungsform der erfindungsgemäßen Vorrichtung ist es vorgesehen, dass der Nasenabschnitt (an oder) durch zwei Äste(n) der Halterung ausgebildet ist, wobei die Äste an einem von dem Steg abweisenden Ende jeweils eine Verbreiterung aufweisen. Bevorzugt können die Äste gegenüber einem Stamm der Halterung geneigt sind, wobei der Stamm zwischen Steg und den Ästen verläuft.

Durch die Ausbildung zweier Äste wird eine individuelle Versorgung eines jeden Nasenloches ermöglicht, wodurch die Vorrichtung noch benutzerfreundlicher ausgeführt und die Handhabung weiter vereinfacht werden kann. Denkbar ist aber auch, dass die Halterung keine Gabelung und somit auch keine Äste aufweist, sondern beispielsweise so ausgebildet ist, dass beide Nasenlöcher durch ein gemeinsames Element der Vorrichtung (oder ein mit der Vorrichtung verbundenes Element) verschlossen werden können.

Die Verbreiterung am Ende der Halterung bzw. der Äste kann der Ausbildung jeweils eines Formstückes dienen, das zum Verschließen jeweils eines Nasenloches geeignet ist. Insbesondere kann die Verbreiterung derart ausgeformt sein, dass diese nur abschnittsweise in den jeweiligen Nasenvorhof eingeführt werden kann und das jeweilige Nasenloch (umfangsseitig) verschließt. Auch andere Formen der Verbreiterung sind denkbar. Zudem kann die Verbreiterung auch so ausgeformt sein, dass diese nicht zum Verschließen eines Nasenloches geeignet ist, sondern lediglich der Befestigung eines entsprechenden Verschlusselementes an der Halterung dient. Ein entsprechender Nasenstopfen ist weiter unten im Detail beschrieben.

Gemäß einer bevorzugten Ausführungsform der erfindungsgemäßen Vorrichtung ist es vorgesehen, dass der Steg zumindest einen ersten Längsabschnitt und einen zweiten Längsabschnitt aufweist, wobei erster Längsabschnitt und zweiter Längsabschnitt entgegengesetzt gekrümmt ausgebildet sind.

Dadurch lässt sich die Vorrichtung in ergonomischer Hinsicht optimal gestalten. Insbesondere lässt sich der gewünschte Druckausgleich auf besonders einfache Weise herbeiführen, wenn der erste Längsabschnitt, welcher erste Längsabschnitt den Beißabschnitt umfasst, linksgekrümmt ausgeführt ist (also eine positive Krümmung aufweist) und der zweite Längsabschnitt, welcher zweite Längsabschnitt den Betätigungsabschnitt umfasst, rechtsgekrümmt ausgeführt ist (also eine negative Krümmung aufweist).

Gemäß einer bevorzugten Ausführungsform der erfindungsgemäßen Vorrichtung ist es vorgesehen, dass das erste Rückhalteelement an einer von dem Nasenabschnitt abgewandten Unterseite des Steges angeordnet ist.

Hierdurch lässt sich die erforderliche Bewegungsabfolge noch effizienter über die erfindungsgemäße Vorrichtung auf den Kiefer und die Nase des Benutzers übertragen.

Gemäß einer bevorzugten Ausführungsform der erfindungsgemäßen Vorrichtung ist es vorgesehen, dass der Beißabschnitt zur klemmenden Aufnahme zwischen der oberen Zahnreihe und der unteren Zahnreihe des Benutzers ausgebildet ist.

Obgleich es nicht notwendig ist, dass der Benutzer den Beißabschnitt mit Zähnen sowohl der unteren als auch der oberen Zahnreihe kontaktiert, erleichtert eine solche Ausbildung die Verwendung der erfindungsgemäßen Vorrichtung ungemein. In einem besonders einfachen Fall kann der Beißabschnitt durch einen schmalen und/oder ebenen Steg ausgebildet sein, dessen Dicke etwa 1-3 mm, vorzugsweise etwa 2 mm, und dessen Breite etwa 0,5-2 cm, vorzugsweise etwa 1 cm, beträgt. Dabei kann der Steg (im Beißanschnitt), abgesehen von dem ersten Rückhalteelement und gegebenenfalls weiteren Rückhalteelementen, frei von Vorsprüngen und/oder Vertiefungen, Nuten und dergleichen ausgebildet sein. Die bestimmungsgemäße Positionierung der Vorrichtung zwischen den Zähnen der oberen und der unteren Zahnreihe des Benutzers kann sich dabei aus der Anordnung des ersten Rückhalteelementes ergeben, welches Rückhalteelement bei bestimmungsgemäßer Verwendung der Vorrichtung als Anschlag der Vorrichtung mit der unteren Zahnreihe in Kontakt bringbar sein sollte, um eine Führung der unteren Zahnreihe vor die obere Zahnreihe zu ermöglichen. Demnach sollte die Vorrichtung bestimmungsgemäß so zwischen oberer und unterer Zahnreihe eingeklemmt werden, dass sich das erste Rückhalteelement hinter der unteren Zahnreihe (also zwischen unterer Zahnreihe und Zunge des Benutzers) befindet.

Bevorzugt weist der Beißabschnitt an der dem ersten Rückhalteelement gegenüberliegenden Seite, also an jener Seite, die von Zähnen der oberen Zahnreihe kontaktiert wird, keine Hindernisse auf, die einer Relativbewegung zwischen oberer Zahnreihe und Vorrichtung bzw. Beißabschnitt entgegenstehen könnten. Um die für die Ausführung des Druckausgleichs gewünschte Relativposition von Unter- und Oberkiefer herstellen zu können, ist es besonders bevorzugt, wenn sich die untere Zahnreihe bzw. der Unterkiefer gemeinsam mit der Vorrichtung bzw. mit dem Beißabschnitt bewegt, während die Position bzw. Stellung der oberen Zahnreihe und damit des Oberkiefers durch die Bewegung der Vorrichtung nicht beeinflusst oder verändert wird.

Gemäß einer bevorzugten Ausführungsform der erfindungsgemäßen Vorrichtung ist es vorgesehen, dass das erste Rückhalteelement in einem Betriebszustand der Vorrichtung, in welchem Betriebszustand der Beißabschnitt zwischen oberer und unterer Zahnreihe des Benutzers gehalten wird und der Benutzer seine Nase durch Betätigung des Betätigungsabschnittes mittels (bzw. mit Hilfe) des Nasenabschnittes verschließt, eine Relativbewegung zwischen der unteren Zahnreihe des Benutzers und dem Beißabschnitt verhindert. Damit ist insbesondere jene Relativbewegung gemeint, die die untere Zahnreihe ausführen müsste, um wieder in ihre Normalstellung zu gelangen, also eine Bewegung in Richtung der Zunge des Benutzers.

Somit überträgt das Rückhalteelement eine Positionsveränderung der Vorrichtung direkt auf die untere Zahnreihe des Benutzers. In anderen Worten: Das Rückhalteelement hält die untere Zahnreihe in einer - gegenüber der Normalstellung - ausgelenkten Stellung. In dieser ausgelenkten Stellung können die Unterkieferfrontzähne vor den Oberkieferfrontzähnen (oder zumindest näher an den Oberkieferfrontzähnen als in der Normalstellung) positioniert sein. Bei Benutzern mit einem Unterbiss - also bei Benutzern, bei denen die Unterkieferfrontzähne in der Normalstellung bereits vor den Oberkieferfrontzähnen liegen - können die Unterkieferfrontzähne durch die erfindungsgemäße Vorrichtung noch weiter ausgelenkt werden, um den Unterkiefer in die gemäß Edmonds-Technik angestrebte ausgelenkte Stellung zu überführen.

Gemäß einer bevorzugten Ausführungsform der erfindungsgemäßen Vorrichtung ist es vorgesehen, dass der Beißabschnitt zumindest eine obere Auflagefläche für die obere Zahnreihe des Benutzers, insbesondere für die Oberkieferfrontzähne, und zumindest eine untere Auflagefläche für die untere Zahnreihe des Benutzers, insbesondere für die Unterkieferfrontzähne, aufweist, wobei das erste Rückhalteelement an der unteren Auflagefläche angeordnet oder durch diese ausgebildet ist.

Hierdurch lässt sich ein besonders einfacher und funktionszuverlässiger Aufbau der erfindungsgemäßen Vorrichtung realisieren. Die Auflageflächen ermöglichen zudem eine besonders stabile und sichere Lagerung der Zähne an der Vorrichtung bzw. am Beißabschnitt, wodurch die Handhabung verbessert bzw. erleichtert und Verletzungen vorgebeugt wird.

Gemäß einer bevorzugten Ausführungsform der erfindungsgemäßen Vorrichtung ist es vorgesehen, dass das erste Rückhalteelement durch einen von der unteren Auflagefläche abstehenden oder an der unteren Auflagefläche ausgeformten Vorsprung ausgebildet ist.

Dabei kann sich der Vorsprung im Wesentlichen transversal zu einer Längsrichtung des Beißabschnittes und/oder des Steges erstrecken, um als Anschlag für die Unterkiefer(front)zähne des Benutzers zu fungieren. Bevorzugt kann der Vorsprung dabei selbst stegförmig ausgebildet sein und sich über die gesamte Breite des Beißabschnittes bzw. des Steges erstrecken. Dadurch wird die Führung des Unterkiefers in die gewünschte Stellung noch sicherer.

Gemäß einer bevorzugten Ausführungsform der erfindungsgemäßen Vorrichtung ist es vorgesehen, dass der Beißabschnitt zumindest ein weiteres Rückhalteelement aufweist, welches weitere Rückhalteelement von dem ersten Rückhalteelement beabstandet ist.

Dadurch wird es möglich, die Vorrichtung zur Verwendung im Zusammenhang mit verschiedenen Gesichts- bzw. Kiefergrößen anbieten zu können. Der jeweilige Benutzer kann jenes Rückhalteelement als Anschlag für seine Unterkiefer(front)zähne auswählen, bei welchem Rückhalteelement sich der Nasenabschnitt besonders nahe an seiner Nase bzw. seinen Nasenlöchern befindet, ohne jedoch in der Ausgangsposition, in welcher Ausgangsposition der Benutzer die Vorrichtung zwar bereits zwischen den Oberkiefer(front)zähnen und den Unterkiefer(front)zähnen hält aber noch keine Kraft auf den Betätigungsabschnitt ausübt, unangenehm auf die Nase oder die Oberlippe des Benutzers zu drücken. Insgesamt kann durch die Anordnung mehrerer Rückhalteelemente im bzw. am Beißabschnitt eine Verwendung der Vorrichtung für unterschiedliche Kiefer- bzw. Gesichtsgrößen und -formen ermöglicht werden.

Gemäß einer bevorzugten Ausführungsform der erfindungsgemäßen Vorrichtung ist es vorgesehen, dass ein Abstand zwischen dem ersten Rückhalteelement und dem weiteren Rückhalteelement zwischen 3 mm und 6 mm, insbesondere zwischen 3,5 mm und 5,5 mm, besonders bevorzugt zwischen 4 mm und 5 mm, beträgt.

Der Abstand zwischen dem ersten Rückhalteelement und dem weiteren Rückhalteelement (bzw., bei Ausführungsformen mit mehr als zwei Rückhalteelementen, der Abstand zwischen benachbarten Rückhalteelementen) soll dabei so gewählt sein, dass (Front-)Zähne des Unterkiefers (gerade noch) zwischen zwei benachbarten Rückhalteelementen Platz finden, sodass deren Schneidekante (Inzisalkante) die untere Auflagefläche des Steges und deren zungenseitige Innenseite das jeweilige Rückhalteelement kontaktieren kann.

Gemäß einer bevorzugten Ausführungsform der erfindungsgemäßen Vorrichtung ist es vorgesehen, dass das erste Rückhalteelement und das weitere Rückhalteelement durch, vorzugsweise parallele, Vorsprünge ausgebildet sind, welche Vorsprünge von der unteren Auflagefläche abstehen und sich in eine quer, vorzugsweise orthogonal, zu einer Längsrichtung des Steges verlaufende Richtung erstrecken.

Auf diese Weise kann eine sichere Führung des Unterkiefers für verschiedene Kiefer- bzw. Gesichtsgrößen und -formen sichergestellt werden.

Gemäß einer bevorzugten Ausführungsform der erfindungsgemäßen Vorrichtung ist es vorgesehen, dass der Nasenabschnitt zumindest einen, vorzugsweise zwei, Nasenstopfen umfasst, oder zur, vorzugsweise kraftschlüssigen, Aufnahme eines Nasenstopfens ausgebildet ist.

Die Nasenstopfen haben dabei die Aufgabe, die Nasenlöcher auf zuverlässige Weise zu verschließen, ohne die Nase des Benutzers zu verletzen. Insbesondere soll das Verletzungsrisiko im Falle zu hoher Kraftausübung auf den Betätigungsabschnitt minimiert sein. Zu diesem Zweck kann der Nasenabschnitt der Vorrichtung einen oder zwei - denkbar sind grundsätzlich auch mehrere - Nasenstopfen umfassen, wobei die Nasenstopfen grundsätzlich aus einem anderen Material gefertigt sein können als der Rest des Nasenabschnittes und/oder der Rest der Vorrichtung. Insbesondere können die Nasenstopfen aus einem elastisch verformbaren, hautverträglichen Kunststoff gefertigt sein, wobei eine Verformung zum Zwecke der Anpassung an unterschiedliche Nasengrößen bzw. -formen durchaus möglich sein soll. Das Material der Nasenstopfen soll dabei nicht cytotoxisch sein. Die Form kann grundsätzlich beliebig gewählt werden, solange ein zuverlässiges Verschließen des der Nasenlöcher möglich ist. Bevorzugt können die Nasenstopfen jedoch bohnenförmig oder kugelförmig ausgebildet sein. Die Nasenstopfen können entweder als Teil der Vorrichtung ausgebildet, oder lösbar mit der Vorrichtung, insbesondere mit dem Nasenabschnitt, verbunden sein.

Gemäß einer bevorzugten Ausführungsform der erfindungsgemäßen Vorrichtung ist vorgesehen, dass der Nasenstopfen eine Shore-A-Härte von 8 bis 17, vorzugsweise von 10 bis 15, aufweist (nach DIN ISO 48-4: 2021).

Durch diese Wahl der Härte ist ein besonders zuverlässiger Verschluss der Nasenlöcher gewährleistet; gleichzeitig können sich die Nasenstopfen aber auch besonders gut an unterschiedliche Nasengrößen anpassen.

Gemäß einer bevorzugten Ausführungsform der erfindungsgemäßen Vorrichtung ist vorgesehen, dass der Nasenstopfen aus, vorzugsweise hypoallergenem, Silikon gefertigt ist und/oder dass der Steg und/oder die Halterung aus, vorzugsweise hypoallergenem, Polypropylen gefertigt ist bzw. sind.

Dadurch ist eine besonders gute Hautverträglichkeit gewährleistet.

Gemäß einer bevorzugten Ausführungsform der erfindungsgemäßen Vorrichtung ist vorgesehen, dass der Betätigungsabschnitt einen Griff umfasst oder durch einen Griff ausgebildet ist.

Hierdurch wird die Handhabung der Vorrichtung noch weiter vereinfacht.

Eine Aufgabe der Erfindung wird auch gelöst durch ein System umfassend eine Vorrichtung gemäß einer der oben erwähnten Ausführungsformen sowie zumindest einen, bevorzugt zwei, Nasenstopfen, wobei der oder die Nasenstopfen mit der Vorrichtung verbunden ist bzw. sind.

Die obigen, die erfindungsgemäße Vorrichtung betreffenden Ausführungen treffen sinngemäß auch auf das erfindungsgemäße System zu. Insbesondere ergeben sich die oben beschriebenen Vorteile in analoger Weise auch bei dem erfindungsgemäßen System.

Die oben beschriebenen Ausführungsformen oder die beschriebenen Merkmale der einzelnen Ausführungsformen sind, soweit nicht anders angegeben, mit anderen Ausführungsformen oder mit einzelnen Merkmalen anderer Ausführungsformen kombinierbar.

### Kurze Beschreibung der Figuren

Im Folgenden werden bevorzugte Ausführungsvarianten der Erfindung anhand der Zeichnungen näher beschrieben. Die folgenden Ausführungen sollen den Erfindungsgedanken aber weder einengen noch abschließend wiedergeben. Es zeigen:
- Fig. 1: eine schematische Darstellung einer erfindungsgemäßen Vorrichtung in einer Seitenansicht,
- Fig. 2: eine schematische Darstellung der Vorrichtung aus Fig. 1 in einer Vorderansicht,
- Fig. 3: eine schematische Darstellung der Vorrichtung aus Fig. 1 in einer Rückansicht,
- Fig. 4: eine schematische Darstellung der Vorrichtung aus Fig. 1 in einer Draufsicht,
- Fig. 5: eine schematische Darstellung eines Paares erfindungsgemäßer Nasenstopfen in einer Vorderansicht,
- Fig. 6: eine schematische Darstellung der Nasenstopfen aus Fig. 5 in einer Draufsicht,
- Fig. 7: eine schematische Darstellung des Nasenstopfens in einer Schnittansicht gemäß A-A aus Fig. 5,
- Fig. 8: eine schematische Darstellung der Nasenstopfen aus Fig. 5 in einer Seitenansicht, sowie
- Fig. 9: eine schematische Darstellung einer Drehung der Vorrichtung.

### Wege zur Ausführung der Erfindung

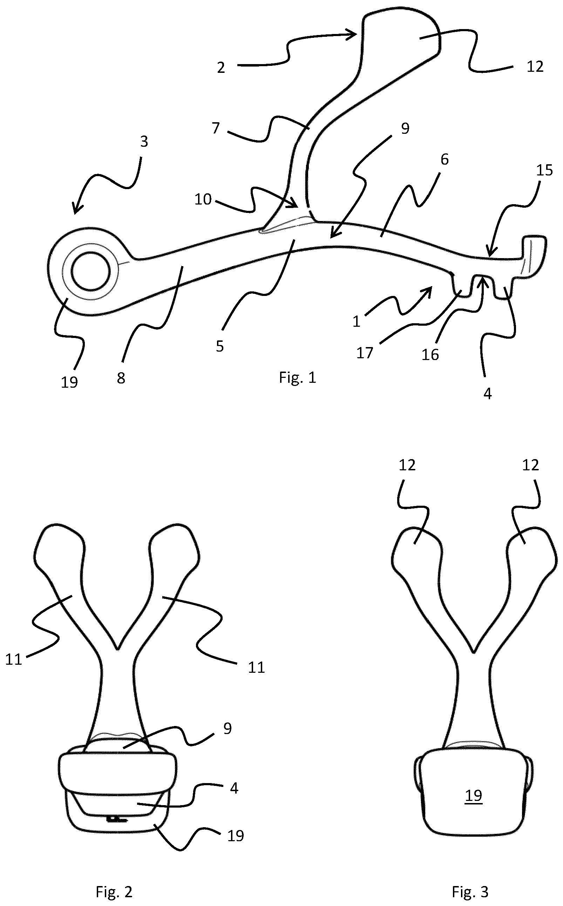

Fig. 1 zeigt die erfindungsgemäße Vorrichtung in einer Seitenansicht. In der gezeigten Ausführungsvariante ist die Vorrichtung als gabelartig ausgebildeter Hebel ausgeformt, wobei ein Beißabschnitt 1 an einem ersten Hebelarm 6, ein Nasenabschnitt 2 an einem zweiten Hebelarm 7 und ein Betätigungsabschnitt 3 an einem dritten Hebelarm 8 angeordnet ist. Die drei Hebelarme 6, 7, 8 sind über eine Gabelung 5 kräfteübertragend miteinander verbunden.

Der Beißabschnitt 1 dient dazu, von einem Benutzer zwischen Oberkieferfrontzähnen und Unterkieferfrontzähnen geklemmt bzw. gehalten zu werden, indem der Benutzer die Vorrichtung mit dem Beißabschnitt 1 voran in seine Mundhöhle einführt und auf den Beißabschnitt beißt. Dabei sollen die Oberkieferfrontzähne eine obere Auflagefläche 15 des Beißabschnittes 1 und die Unterkieferfrontzähne eine untere Auflagefläche 16 des Beißabschnittes kontaktieren. Zusätzlich sollen die Unterkieferfrontzähne so am Beißabschnitt 1 positioniert werden, dass ein erstes Rückhalteelement 4 zwischen den Unterkieferfrontzähnen und der Zunge des Benutzers positioniert ist oder das erste Rückhalteelement 4 an einer zungenseitigen Innenseite der Unterkieferfrontzähne anliegt. In der gezeigten Ausführungsvariante, gemäß welcher der Beißabschnitt 1 auch noch ein weiteres Rückhalteelement 17 aufweist, wären die Unterkieferfrontzähne demnach zwischen dem ersten Rückhalteelement 4 und dem weiteren Rückhalteelement 17 zu positionieren. Um die Vorrichtung bestimmungsgemäß zwischen unterer und oberer Zahnreihe zu halten, reicht es aus, locker auf den Beißabschnitt 1 zu beißen.

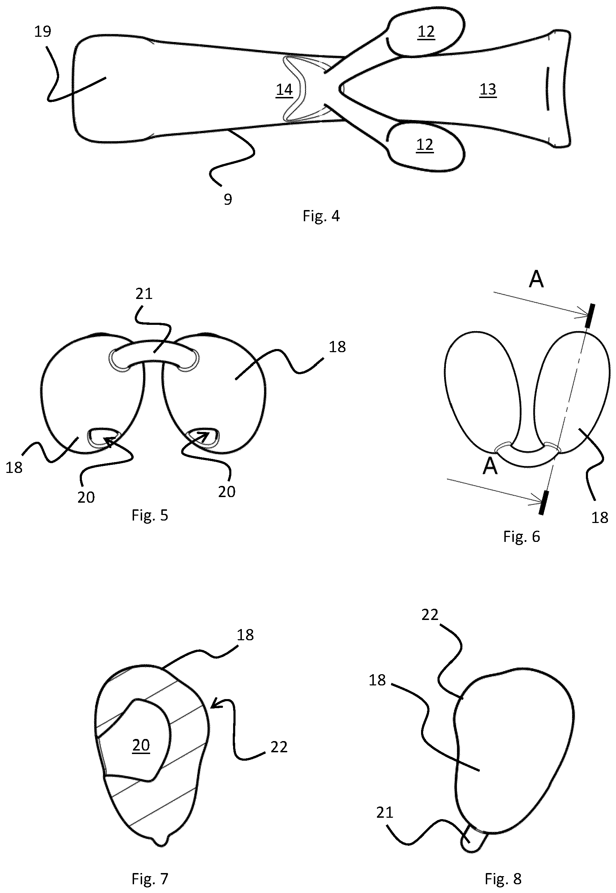

Durch die gabelartige Ausbildung der Vorrichtung befindet sich der Nasenabschnitt 2 automatisch in der Nähe der Nasenlöcher des Benutzers, sobald dieser die Vorrichtung bestimmungsgemäß zwischen oberer und unterer Zahnreihe, insbesondere zwischen den Oberkieferfrontzähnen und den Unterkieferfrontzähnen, eingeklemmt hat. Dabei setzt eine Halterung 10 etwa mittig an einem (im Wesentlichen horizontal verlaufenden) Steg 9 an, um sich zunächst in eine im Wesentlichen vertikale Richtung (weg vom Steg 9) und dann mit einer leichten Biegung in Richtung des Beißabschnittes 1 zu erstrecken. Dadurch ergibt sich der gabelförmige (oder Y-förmige) Hebel. An einem vom Steg 9 abgewandten Ende der Halterung 10 ist eine Verbreiterung 12 ausgeformt, welche Verbreiterung 12 in der gezeigten Ausführungsvariante den Nasenabschnitt 2 ausbildet und zur lösbaren Befestigung von Nasenstopfen 18 dient (siehe Fig. 5, 6, 7 und 8).

Wie aus den Fig. 2, 3 und 4 zu erkennen ist, umfasst die Halterung 10 der gezeigten Ausführungsvariante zwei Äste 11, die sich in etwa der halben Länge des zweiten Hebelarmes 7 bzw. der Halterung 10 verzweigen. Dabei weist jeder Ast 11 an einem vom Steg abgewandten Ende eine eigene Verbreiterung 12 auf, um jeweils einen Nasenstopfen 18 aufzunehmen.

Die Fig. 5, 6 und 8 zeigen jeweils ein Paar miteinander verbundener Nasenstopfen 18, wobei die Nasenstopfen 18 bezüglich einer durch die Verbindung 21 und zwischen den Nasenstopfen 18 verlaufenden Symmetrieebene spiegelsymmetrisch ausgebildet sind. Dabei weisen die Nasenstopfen 18 im Wesentlichen die Form einer Bohne (triaxiales Ellipsoid) auf, wobei jedoch an einer Unterseite des Nasenstopfens 18 jeweils eine Öffnung 20 und an einer dieser Unterseite gegenüberliegenden Oberseite des Nasenstopfens 18 eine Erhebung 22 ausgeformt ist.

Wie aus Fig. 7 zu erkennen ist, entspricht eine Form der Öffnung 20 der Form der Verbreiterung 12, die den jeweiligen Nasenabschnitt 2 ausbildet und zur Verbindung der Vorrichtung mit dem jeweiligen Nasenstopfen 18 dient. Dabei kann die Öffnung 20 (bzw. das durch die Öffnung 20 freigegebene Innenvolumen) zur Aufnahme des zugeordneten Nasenabschnittes 2 analog zum jeweiligen Nasenabschnitt 2 bzw. der jeweiligen Verbreiterung 12 asymmetrisch ausgebildet sein, um eine Vorzugsorientierung der mit der Vorrichtung zu verbindenden Nasenstopfen 18 zu definieren.

Aus der Zusammenschau von Fig. 4 und Fig. 1 ist besonders gut ersichtlich, dass der Steg 9 einen in unterschiedliche Richtungen gekrümmten Längsverlauf aufweist. Dabei weist ein erster Längsabschnitt 13, in welchem sich der Bissabschnitt 1 befindet, eine positive Krümmung und ein zweiter Längsabschnitt 14, von welchem zweiten Längsabschnitt 14 die Halterung 10 abragt und der darüber hinaus auch den Betätigungsabschnitt 3 umfasst, eine negative Krümmung auf.

In der gezeigten Ausführungsvariante ist der Betätigungsabschnitt 3 durch einen Griff 19 ausgebildet. An diesem Griff 19 kann der Benutzer die Vorrichtung mit Daumen und Zeigefinger halten und insbesondere nach oben und unten bewegen.

Ist nun die Vorrichtung bestimmungsgemäß über den Beißabschnitt 1 zwischen den Oberkieferfrontzähnen und den Unterkieferfrontzähnen des Benutzers eingeklemmt und sind die Nasenstopfen 18 darüber hinaus bestimmungsgemäß über die Verbreiterungen 12 des Nasenabschnittes 2 lösbar mit der Vorrichtung verbunden, so führt ein Anheben des Betätigungsabschnittes 3 dazu, dass sich die Nasenstopfen 18 weiter in Richtung der Nasenlöcher des Benutzers bewegen. Sobald die Nasenstopfen 18 die Nasenlöcher erreicht haben, dringen die Nasenstopfen 18, insbesondere mit ihren Erhebungen 22, in den Nasenvorhof des jeweiligen Nasenloches ein; in weiterer Folge führt ein weiteres Anheben des Betätigungsabschnittes 3 dazu, dass die Nasenlöcher durch die Nasenstopfen 18 verschlossen werden - mit steigendem Anpressdruck steigt auch die Zuverlässigkeit des Verschlusses der Nasenlöcher durch die Nasenstopfen 18.

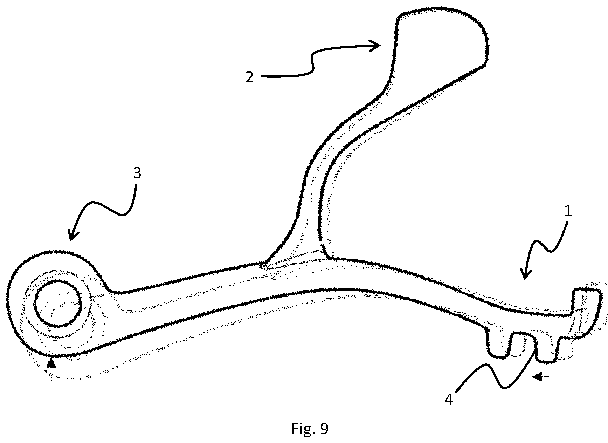

Sobald keine (wesentliche) Verformung der Nase und/oder der Nasenstopfen 18 mehr stattfindet, führt ein weiteres Anheben des Betätigungsabschnittes 3 im Wesentlichen zu einer Rotation der Vorrichtung um den Kontaktbereich, in dem die Nasenstopfen 18 an die Nasenlöcher gepresst werden, bzw. um den Nasenabschnitt 2 (Fig. 9). Diese Drehbewegung führt im Bereich des Beißabschnittes 1 zu einer Veränderung der Position des ersten Rückhalteelementes 4, wodurch sich auch die Stellung der unteren Zahnreihe - und damit auch die Position des Unterkiefers - gegenüber dem (stationär gebliebenen) Oberkiefer verschiebt. Atmet der Benutzer nun gegen die Nasenstopfen 18, die seine Nasenlöcher aufgrund des aufrechterhaltenen Anpressdruckes nach wie vor fest verschließen, kommt es zu dem gewünschten Druckausgleich im Mittelohr.

Da der Benutzer durch bestimmungsgemäße Verwendung der erfindungsgemäßen Vorrichtung den komplexen Bewegungs- und Atmungsablauf der Edmonds-Technik durchführt, kann ein Druckausgleich reproduzierbar und zuverlässig herbeigeführt werden - selbst bei Vorliegen einer Tubenbelüftungsstörung. Zudem ermöglicht die erfindungsgemäße Vorrichtung das Erlernen und Trainieren dieser komplexen, aber zuverlässigen Druckausgleichstechnik ohne die Verwendung der Vorrichtung - beispielsweise unter Wasser.

### Bezugszeichenliste

- 1: Beißabschnitt
- 2: Nasenabschnitt
- 3: Betätigungsabschnitt
- 4: erstes Rückhalteelement
- 5: Gabelung
- 6: erster Hebelarm
- 7: zweiter Hebelarm
- 8: dritter Hebelarm
- 9: Steg
- 10: Halterung
- 11: Ast
- 12: Verbreiterung
- 13: erster Längsabschnitt
- 14: zweiter Längsabschnitt
- 15: obere Auflagefläche
- 16: untere Auflagefläche
- 17: weiteres Rückhalteelement
- 18: Nasenstopfen
- 19: Griff
- 20: Öffnung
- 21: Verbindung
- 22: Erhebung

## Patentansprüche

1. Vorrichtung zur Durchführung eines Druckausgleichs zwischen einem Mittelohr eines Benutzers und einer Umgebung des Mittelohrs, wobei die Vorrichtung zumindest
a. einen Beißabschnitt (1) zur Aufnahme zumindest eines Abschnitts einer unteren Zahnreihe des Benutzers, wobei der Beißabschnitt (1) vorzugsweise zur klemmenden Aufnahme zwischen einer oberen Zahnreihe und der unteren Zahnreihe des Benutzers ausgebildet ist, zumindest
b. einen Nasenabschnitt (2) zum Verschließen einer Nase des Benutzers sowie zumindest
c. einen Betätigungsabschnitt (3) zur manuellen Betätigung der Vorrichtung durch den Benutzer zur Durchführung des Druckausgleichs aufweist,
wobei Beißabschnitt (1), Nasenabschnitt (2) und Betätigungsabschnitt (3) voneinander beabstandet und kräfteübertragend miteinander verbunden sind, und wobei der Beißabschnitt (2) zumindest ein erstes Rückhalteelement (4) aufweist, um einen Anschlag für die untere Zahnreihe des Benutzers auszubilden.

2. Vorrichtung nach Anspruch 1, **dadurch gekennzeichnet, dass** Beißabschnitt (1), Nasenabschnitt (2) und Betätigungsabschnitt (3) über eine Gabelung (5) miteinander verbunden sind.

3. Vorrichtung nach einem der Ansprüche 1 oder 2, **dadurch gekennzeichnet, dass** die Vorrichtung einen gabelartig ausgebildeten Hebel umfasst oder als gabelartiger Hebel ausgebildet ist, wobei der Beißabschnitt (1) an einem ersten Hebelarm (6), der Nasenabschnitt (2) an einem zweiten Hebelarm (7) und der Betätigungsabschnitt (3) an einem dritten Hebelarm (8) angeordnet ist.

4. Vorrichtung nach einem der Ansprüche 1 bis 3, **dadurch gekennzeichnet, dass** die Vorrichtung einen Steg (9) umfasst, wobei Beißabschnitt (1) und Betätigungsabschnitt (3) durch jeweils einen Abschnitt, vorzugsweise Endabschnitt, des Steges (9) ausgebildet sind, wobei vorzugsweise das erste Rückhalteelement (4) an einer von dem Nasenabschnitt (2) abgewandten Unterseite des Steges (9) angeordnet ist.

5. Vorrichtung nach Anspruch 4, **dadurch gekennzeichnet, dass** die Vorrichtung eine Halterung (10) umfasst, welche Halterung (10) von dem Steg (9) abragt und den Nasenabschnitt (2) aufweist, wobei vorzugsweise der Nasenabschnitt (2) durch zwei Äste (11) der Halterung (10) ausgebildet ist, wobei die Äste (11) an einem von dem Steg (9) abweisenden Ende jeweils eine Verbreiterung (12) aufweisen.

6. Vorrichtung nach einem der Ansprüche 4 oder 5, **dadurch gekennzeichnet, dass** der Steg (9) zumindest einen ersten Längsabschnitt (13) und einen zweiten Längsabschnitt (14) aufweist, wobei erster Längsabschnitt (13) und zweiter Längsabschnitt (14) entgegengesetzt gekrümmt ausgebildet sind.

7. Vorrichtung nach einem der Ansprüche 1 bis 6, **dadurch gekennzeichnet, dass** das erste Rückhalteelement (4) in einem Betriebszustand der Vorrichtung, in welchem Betriebszustand der Beißabschnitt (1) zwischen oberer und unterer Zahnreihe des Benutzers gehalten wird und der Benutzer seine Nase durch Betätigung des Betätigungsabschnittes (3) mittels des Nasenabschnittes (2) verschließt, eine Relativbewegung zwischen der unteren Zahnreihe des Benutzers und dem Beißabschnitt (1) verhindert.

8. Vorrichtung nach einem der Ansprüche 1 bis 7, **dadurch gekennzeichnet, dass** der Beißabschnitt (1) zumindest eine obere Auflagefläche (15) für eine obere Zahnreihe des Benutzers und zumindest eine untere Auflagefläche (16) für die untere Zahnreihe des Benutzers aufweist, wobei das erste Rückhalteelement (4) an der unteren Auflagefläche (16) angeordnet oder durch diese ausgebildet ist, wobei vorzugsweise das erste Rückhalteelement (4) durch einen von der unteren Auflagefläche (16) abstehenden Vorsprung ausgebildet ist.

9. Vorrichtung nach einem der Ansprüche 1 bis 8, **dadurch gekennzeichnet, dass** der Beißabschnitt (1) zumindest ein weiteres Rückhalteelement (17) aufweist, welches weitere Rückhalteelement (17) von dem ersten Rückhalteelement (4) beabstandet ist, wobei ein Abstand zwischen dem ersten Rückhalteelement (4) und dem weiteren Rückhalteelement (17) vorzugsweise zwischen 3 mm und 6 mm, insbesondere zwischen 3,5 mm und 5,5 mm, besonders bevorzugt zwischen 4 mm und 5 mm, beträgt.

10. Vorrichtung nach Anspruch 9, **dadurch gekennzeichnet, dass** das erste Rückhalteelement (4) und das weitere Rückhalteelement (17) durch, vorzugsweise parallele, Vorsprünge ausgebildet sind, welche Vorsprünge von der unteren Auflagefläche (16) abstehen und sich in eine quer, vorzugsweise orthogonal, zu einer Längsrichtung des Steges (9) verlaufende Richtung erstrecken.

11. Vorrichtung nach einem der Ansprüche 1 bis 10, **dadurch gekennzeichnet, dass** der Nasenabschnitt (2) zumindest einen, vorzugsweise zwei, Nasenstopfen (18) umfasst, oder zur, vorzugsweise kraftschlüssigen, Aufnahme eines Nasenstopfens (18) ausgebildet ist, wobei der Nasenstopfen (18) vorzugsweise eine Shore-A-Härte von 8 bis 17, vorzugsweise von 10 bis 15, aufweist.

12. Vorrichtung nach Anspruch 11, **dadurch gekennzeichnet, dass** der Nasenstopfen (18) aus, vorzugsweise hypoallergenem, Silikon gefertigt ist.

13. Vorrichtung nach einem der Ansprüche 4 bis 12, **dadurch gekennzeichnet, dass** der Steg (9) und/oder die Halterung (10) aus, vorzugsweise hypoallergenem, Polypropylen gefertigt ist bzw. sind.

14. Vorrichtung nach einem der Ansprüche 1 bis 13, **dadurch gekennzeichnet, dass** der Betätigungsabschnitt (3) einen Griff umfasst oder durch einen Griff ausgebildet ist.

15. System umfassend eine Vorrichtung nach einem der Ansprüche 1 bis 14 sowie zumindest einen Nasenstopfen (18), wobei der Nasenstopfen (18) mit der Vorrichtung verbunden ist.

## Claims

1. Device for performing pressure equalization between a user's middle ear and the environment of the middle ear, wherein the device comprises at least:
a. a biting section (1) for receiving at least a portion of a lower row of teeth of the user, preferably designed for clamping engagement between an upper and lower row of teeth of the user,
b. a nose section (2) for sealing a nose of the user, and
c. an actuation section (3) for manual operation of the device by the user to perform pressure equalization,
wherein the biting section (1), nose section (2), and actuation section (3) are spaced apart and connected to each other in a force-transmitting manner, and wherein the biting section (1) comprises at least a first retaining element (4) to form a stop for the user's lower row of teeth.

2. Device according to claim 1, **characterized in that** the biting section (1), nose section (2), and actuation section (3) are connected via a fork (5).

3. Device according to one of claims 1 or 2, **characterized in that** the device comprises or is formed as a fork-shaped lever, wherein the biting section (1) is arranged on a first lever arm (6), the nose section (2) on a second lever arm (7), and the actuation section (3) on a third lever arm (8).

4. Device according to one of claims 1 to 3, **characterized in that** the device comprises a web (9), wherein the biting section (1) and actuation section (3) are formed by respective sections, preferably end sections, of the web (9), and wherein the first retaining element (4) is preferably arranged on a bottom side of the web (9) facing away from the nose section (2).

5. Device according to claim 4, **characterized in that** the device comprises a mount (10) which protrudes from the web (9) and comprises the nose section (2), wherein the nose section (2) is preferably formed by two branches (11) of the mount (10), and the branches (11) each have a widening (12) at an end facing away from the web (9).

6. Device according to one of claims 4 or 5, **characterized in that** the web (9) comprises at least a first longitudinal section (13) and a second longitudinal section (14), wherein the first and second longitudinal sections are formed with opposite curvatures.

7. Device according to one of claims 1 to 6, **characterized in that** the first retaining element (4), in an operating state of the device in which the biting section (1) is held between the upper and lower rows of teeth of the user and the user seals their nose by actuating the actuation section (3) via the nose section (2), prevents relative movement between the user's lower row of teeth and the biting section (1).

8. Device according to one of claims 1 to 7, **characterized in that** the biting section (1) comprises at least an upper support surface (15) for the user's upper row of teeth and at least a lower support surface (16) for the user's lower row of teeth, wherein the first retaining element (4) is arranged on or formed by the lower support surface (16), preferably formed by a projection protruding from the lower support surface (16).

9. Device according to one of claims 1 to 8, **characterized in that** the biting section (1) comprises at least one additional retaining element (17) spaced apart from the first retaining element (4), wherein the distance between the first and additional retaining element (17) is preferably between 3 mm and 6 mm, particularly between 3.5 mm and 5.5 mm, and most preferably between 4 mm and 5 mm.

10. Device according to claim 9, **characterized in that** the first and additional retaining elements (4, 17) are formed by projections, preferably parallel, which protrude from the lower support surface (16) and extend in a direction transverse, preferably orthogonal, to a longitudinal direction of the web (9).

11. Device according to one of claims 1 to 10, **characterized in that** the nose section (2) comprises at least one, preferably two nose plugs (18), or is designed to receive a nose plug (18), preferably in a force-fitting manner, wherein the nose plug (18) preferably has a Shore A hardness of 8 to 17, preferably 10 to 15.

12. Device according to claim 11, **characterized in that** the nose plug (18) is made of silicone, preferably hypoallergenic silicone.

13. Device according to one of claims 4 to 12, **characterized in that** the web (9) and/or the mount (10) is/are made of polypropylene, preferably hypoallergenic polypropylene.

14. Device according to one of claims 1 to 13, **characterized in that** the actuation section (3) comprises or is formed by a handle.

15. System comprising a device according to one of claims 1 to 14 and at least one nose plug (18), wherein the nose plug (18) is connected to the device.

## Revendications

1. Dispositif destiné à effectuer une égalisation de pression entre une oreille moyenne d'un utilisateur et l'environnement de cette oreille moyenne, le dispositif comprenant au moins :
a. une section de morsure (1) destinée à recevoir au moins une partie de la rangée inférieure de dents de l'utilisateur, ladite section étant de préférence conçue pour une prise en étau entre la rangée supérieure et inférieure de dents,
b. une section nasale (2) destinée à obturer le nez de l'utilisateur, et
c. une section d'actionnement (3) permettant à l'utilisateur d'actionner manuellement le dispositif pour effectuer l'égalisation de pression,
les sections de morsure (1), nasale (2) et d'actionnement (3) étant espacées les unes des autres et reliées de manière à transmettre des forces, la section de morsure (1) comprenant au moins un premier élément de retenue (4) servant de butée pour la rangée inférieure de dents de l'utilisateur.

2. Dispositif selon la revendication 1, **caractérisé en ce que** les sections de morsure (1), nasale (2) et d'actionnement (3) sont reliées par une fourche (5).

3. Dispositif selon l'une des revendications 1 ou 2, **caractérisé en ce qu'**il comprend ou est formé comme un levier en forme de fourche, la section de morsure (1) étant disposée sur un premier bras de levier (6), la section nasale (2) sur un deuxième bras de levier (7), et la section d'actionnement (3) sur un troisième bras de levier (8).

4. Dispositif selon l'une des revendications 1 à 3, **caractérisé en ce qu'**il comprend une barre (9), les sections de morsure (1) et d'actionnement (3) étant formées par des sections, de préférence des extrémités, de ladite barre (9), le premier élément de retenue (4) étant de préférence disposé sur une face inférieure de la barre (9) opposée à la section nasale (2).

5. Dispositif selon la revendication 4, **caractérisé en ce qu'**il comprend un support (10) s'étendant à partir de la barre (9) et comprenant la section nasale (2), ladite section étant de préférence formée par deux branches (11) du support (10), lesdites branches (11) comportant chacune une élargissement (12) à leur extrémité opposée à la barre (9).

6. Dispositif selon l'une des revendications 4 ou 5, **caractérisé en ce que** la barre (9) comprend au moins une première section longitudinale (13) et une deuxième section longitudinale (14), lesdites sections étant courbées en sens opposés.

7. Dispositif selon l'une des revendications 1 à 6, **caractérisé en ce que** le premier élément de retenue (4), dans un état de fonctionnement du dispositif où la section de morsure (1) est maintenue entre les rangées supérieure et inférieure de dents de l'utilisateur et où l'utilisateur ferme son nez en actionnant la section d'actionnement (3) via la section nasale (2), empêche tout mouvement relatif entre la rangée inférieure de dents et la section de morsure (1).

8. Dispositif selon l'une des revendications 1 à 7, **caractérisé en ce que** la section de morsure (1) comprend au moins une surface de support supérieure (15) pour la rangée supérieure de dents et au moins une surface de support inférieure (16) pour la rangée inférieure, le premier élément de retenue (4) étant disposé sur ou formé par la surface de support inférieure (16), de préférence sous forme d'un proéminence saillante.

9. Dispositif selon l'une des revendications 1 à 8, **caractérisé en ce que** la section de morsure (1) comprend au moins un autre élément de retenue (17) espacé du premier élément de retenue (4), la distance entre les deux étant de préférence comprise entre 3 mm et 6 mm, notamment entre 3,5 mm et 5,5 mm, et idéalement entre 4 mm et 5 mm.

10. Dispositif selon la revendication 9, **caractérisé en ce que** les éléments de retenue (4, 17) sont formés par des proéminences, de préférence parallèles, s'étendant à partir de la surface de support inférieure (16) dans une direction transversale, de préférence orthogonale, à la direction longitudinale de la barre (9).

11. Dispositif selon l'une des revendications 1 à 10, **caractérisé en ce que** la section nasale (2) comprend au moins un, de préférence deux bouchons nasaux (18), ou est conçue pour recevoir un bouchon nasal (18), de préférence par emboîtement, le bouchon nasal (18) ayant de préférence une dureté Shore A de 8 à 17, de préférence de 10 à 15.

12. Dispositif selon la revendication 11, **caractérisé en ce que** le bouchon nasal (18) est fabriqué en silicone, de préférence hypoallergénique.

13. Dispositif selon l'une des revendications 4 à 12, **caractérisé en ce que** la barre (9) et/ou le support (10) est/sont fabriqué(s) en polypropylène, de préférence hypoallergénique.

14. Dispositif selon l'une des revendications 1 à 13, **caractérisé en ce que** la section d'actionnement (3) comprend ou est formée par une poignée.

15. Système comprenant un dispositif selon l'une des revendications 1 à 14 ainsi qu'au moins un bouchon nasal (18), le bouchon nasal (18) étant relié au dispositif.
